Europäisches Patentamt

European Patent Office

Office européen des brevets

(11) Veröffentlichungsnummer: **0 431 400 A1**

(12) **EUROPÄISCHE PATENTANMELDUNG**

(21) Anmeldenummer: 90122257.0

(22) Anmeldetag: 22.11.90

(51) Int. Cl.⁵: **C12Q 1/00**, G01N 33/543,
C12N 11/00, C12N 11/06

(30) Priorität: 02.12.89 DE 3939973

(43) Veröffentlichungstag der Anmeldung:
12.06.91 Patentblatt 91/24

(84) Benannte Vertragsstaaten:
AT BE CH DE DK ES FR GB GR IT LI LU NL SE

(71) Anmelder: BASF Aktiengesellschaft
Carl-Bosch-Strasse 38
W-6700 Ludwigshafen(DE)

(72) Erfinder: Fuchs, Harald, Dr.
Boehlweg 32
W-6719 Carlsberg(DE)
Erfinder: Licht, Ulrike, Dr.
Meerfeldstrasse 62
W-6800 Mainheim 1(DE)
Erfinder: Ringsdorf, Helmut, Prof. Dr.
Kehlweg 41
W-6500 Mainz-Gonsenheim(DE)
Erfinder: Blankenburg, Rainer, Dr.
Bruesseler Ring 45
W-6700 Ludwigshafen(DE)
Erfinder: Ahlers, Michel
Josephstrasse 16
W-6500 Mainz(DE)

(54) Zweidimensional kristallisierte Makromolekülschichten.

(57) Die Erfindung betrifft zweidimensional kristallisierte Makromolekülschichten, welche unter Zuhilfenahme einer Biotineinheiten tragenden lipiden Monoschicht aufgebaut sind.

FIG.1

FIG.2

FIG.3

EP 0 431 400 A1

## ZWEIDIMENSIONAL KRISTALLISIERTE MAKROMOLEKÜLSCHICHTEN

Die Erfindung betrifft zweidimensional kristallisierte Makromolekülschichten, welche unter Zuhilfenahme einer Biotineinheiten tragenden lipiden Monoschicht aufgebaut sind.

Die Ausbildung kristalliner Schichten natürlicher Makromoleküle, insbesondere von Proteinen ist von besonderem Interesse. Solche Schichten erlauben Strukturuntersuchungen dieser Stoffe, die Möglichkeit zur Analyse struktureller Zusammenhänge zwischen den Molekülen sowie gegebenenfalls die Ausarbeitung neuer Anwendungsbereiche. Gerade jedoch interessierende Proteine bilden auf natürlichem Wege keine für die Analyse geeignete Kristalle. Es hat deshalb nicht an Versuchen gefehlt, kristallisierte Proteinschichten zu erzeugen. So berichteten Uzigiris & Kornberg (Nature 301 (1983) 125-129) von einem Verfahren zur Ausbildung zweidimensionaler Proteinschichten auf Lipidschichten. Dieses Verfahren gestattet die spezifische Bindung von Proteinen an natürliche oder synthetische im ebenen Lipidfilm vorhandene Lipidliganden. Trotz der Bereitstellung einer Reihe von kristallisierten Proteinschichten bestand jedoch der Nachteil, daß für jedes Protein ein spezifischer Lipidligand, somit ein Protein-Antigen-Paar, zur Verfügung stehen muß. In diesem Zusammenhang wurde auch die Bildung von speziellen Proteinschichten, denen des Streptavidins, mit Hilfe dieser Technik, vorgeschlagen. Dazu wurde das für die Lipidschicht vorgesehene Lipid mit einem Biotinliganden versehen, so daß sich auf Grund der besonders hohen Affinität des Biotins zum Streptavidin die Streptavidinmoleküle an die Biotinliganden der Lipidschicht anlagern können und im Wasser/Luft-Übergangsbereich eine zweidimensional kristallisierte Streptavidinschicht bilden. Die direkte Beobachtung solcher Schichten gelingt mit Hilfe der Fluoreszenzmikroskopie, der kristalline Charakter kann mittels Elektronenmikroskopie bestimmt werden (Blankenburg et al., Biochemistry 1989, Vol. 28, 8214).

Aufgabe der vorliegenden Erfindung war es somit, kristallisierte Makromolekülschichten bereitzustellen, welche bisher auf einfache Weise nicht zugänglich waren.

Es wurde nun gefunden, daß sich die gestellte Aufgabe mit zweidimensional kristallisierten Makromolekülschichten lösen läßt, welche aus einer ersten, Biotin-Einheiten tragenden lipiden Monoschicht und einer zweiten, an die Biotin-Einheiten sich anlagernde und kristallisierende Schicht aus Streptavidin sowie einer über die freien aktiven Zentren der Streptavidin-Schicht aufgebauten Schicht aus einem Biotin-substituierten Makromolekül bestehen.

Somit bestehen diese kristallisierten Makromolekülschichten aus Biotin-substituierten Makromolekülen, welche an die Streptavidinschicht gebunden sind. In einer weiteren Ausgestaltung der erfindungsgemäßen Schichten wird die Bindung indirekt über ein niedermolekulares oder polymeres Biotin-Ligand-Molekül erreicht. Die Kristallisation der zweiten Makromolekülschicht wird sowohl durch die rezeptoraffine Bindung als auch durch die bereits vorhandene Ordnung der Streptavidinschicht induziert.

In einer besonderen Ausgestaltung der erfindungsgemäßen Schichten bestehen die Makromoleküle aus Proteinen.

Die erste, die erfindungsgemäßen kristallisierten Makromolekülschichten charakterisierende Biotin-Einheiten tragende lipide Monoschicht besteht im allgemeinen aus den bekannten aminofunktionellen Lipiden.

Als besonders vorteilhaft im Sinne der Erfindung haben sich hierbei Verbindungen, wie z.B. N-biotinyl(S-(1,2-bis(octadecyloxycarbonyl)ethyl)-cystein, N-biotinyl-S-1-carboxy-2-((N,N-dioctadecylamino)carbonyl)ethyl)cystein, N-biotinoyl-S-1-phosphatidyl-3-glycero-sn-dimyristoyl, N,N-dioctadecyl-N'-biotinyl-propandiamin und N,N-dioctadecyl-biotinamid erwiesen.

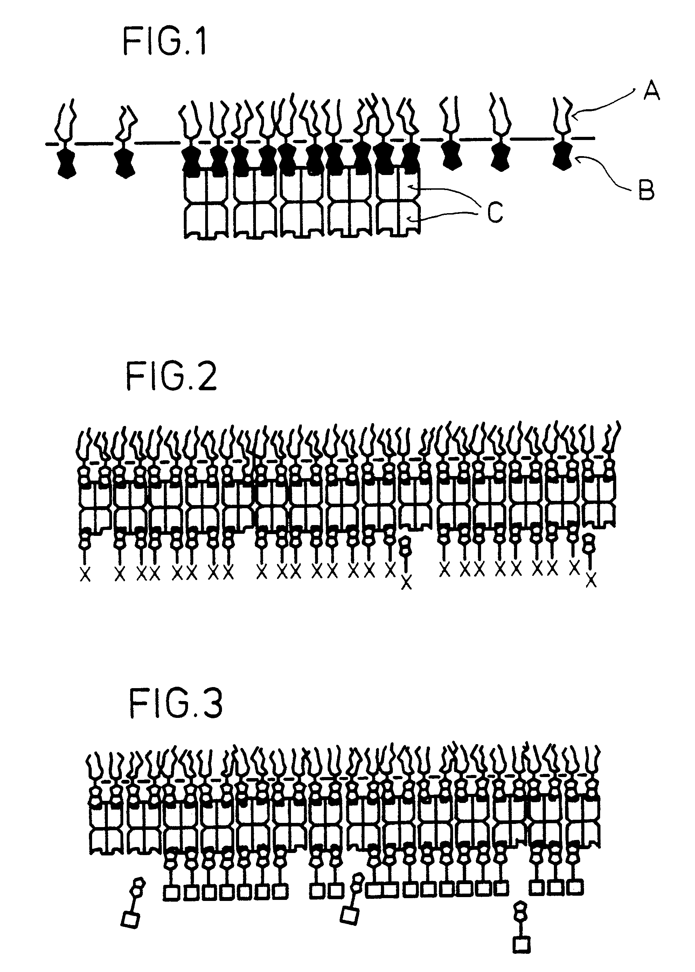

An diese Biotin-substituierte Lipidschicht ist nun eine Streptavidin-Schicht über die spezifischen Rezeptoren angelagert. Streptavidin ist ein Protein, das vier identische Untereinheiten aufweist, wobei jede ein Biotinmolekül binden kann. Die nicht kovalente Bindung zwischen Streptavidin und Biotin ist bekanntermaßen eine der stärksten, der Wert $K_a$ beträgt etwa $10^{15}$ mol$^{-1}$. Durch diese Anlagerung des Streptavidins an die Biotineinheiten der Lipidschicht entsteht eine geordnete, zweidimensionale Schicht, wie sie beispielhaft in Figur 1 mit (A) = Lipid, (B) = Biotin und (C) = Streptavidin dargestellt ist. Auf diese Weise entstehen sehr große zweidimensionale Streptavidin-Bereiche in einer Ausdehnung von 50 bis 200 $\mu$m. Ihr Nachweis ist mit Hilfe der Fluoreszenz-Mikroskopie möglich.

Mit der dargestellten Oberfläche der zweidimensional kristallisierten Streptavidinschicht wird nun die Anlagerung eines Biotin-substituierten, wasserlöslichen Makromoleküls, insbesondere eines Biotin-substituierten Proteins an die freien Anlagerungsstellen der Biotinoberfläche möglich. Solche wasserlöslichen Makromoleküle können Biotin-substituiertes Poly (meth)acrylat, Polylysin, Polyvinylalkohol oder Dextran sein. Sie dienen der Verankerung eines weiteren Ligandmoleküls an die bereits kristallisierte Oberfläche. Proteine, wie z.B. Biotin-substituierte monoklonale Antikörper oder Enzyme können direkt an die Streptavidinschicht gebunden und in überraschend einfacher Weise zu kristallisierten Schichten aufgebaut werden. Die Figuren 2 und 3 zeigen die erfindungsgemäßen Schichten, wobei X allgemein für ein Makromolekül steht und die Quadrate ⊏ das Protein darstellen sollen.

Die derart aufgebauten zweidimensional kristallisierten Makromolekül-Schichten lassen sich dadurch erhalten, daß ein wasserlösliches Biotin-Ligand-Adaptermolekül an die Streptavidinschicht gebunden wird und damit einem weiteren Protein neue Erkennungsstrukturen zur Verfügung stellt. Auch läßt sich ein wasserlösliches Polymer mit Biotin und Ligandstrukturen an die Streptavidinschicht binden, ebenso wie sich ein Biotin-substituiertes Protein an die Streptavidinschicht koppeln läßt. Angaben für die experimentelle Vorgehensweise finden sich u.a. in Blankenburg et al., Biochemistry 1989, Vol. 28, 8214.

Der Nachweis der so erhaltenen Schichten erfolgt in einfacher Weise durch direkte fluoreszenzmikroskopische Beobachtung. Die Kristallinität der erhaltenen Schichten kann durch Elektronenmikroskopie nach Übertragung gemäß der Langmuir-Schäfer-Technik überprüft werden.

Die erfindungsgemäßen kristallisierten Makromolekülschichten erlauben die schnelle und einfache Bereitung von Proben zur Strukturuntersuchung dieser Makromoleküle, welche ansonsten nicht in kristallisierter Form vorliegen. Diese Schichten eignen sich auch zur Kontrolle enzymatischer Reaktionen, wenn der Proteintyp unter Erhalt seiner enzymatischen Aktivität an die Streptavidinschicht angekoppelt ist.

## Ansprüche

Zweidimensional kristallisierte Makromolekülschichten bestehend aus einer ersten, Biotin-Einheiten tragenden lipiden Monoschicht und einer zweiten, an die Biotin-Einheiten sich anlagernde und kristallisierende Schicht aus Streptavidin sowie einer über die freien aktiven Zentren der Streptavidin-Schicht aufgebauten Schicht aus einem Biotin-substituierten Makromolekül.

# FIG.1

# FIG.2

# FIG.3

**Europäisches Patentamt**

## EUROPÄISCHER
## RECHERCHENBERICHT

Nummer der Anmeldung

**EP 90 12 2257**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 116 751  (THE TRUSTEES OF THE LELAND STANFORD JUNIOR UNIVERSITY) <br> * Das ganze Dokument * <br> — — — | 1 | C 12 Q 1/00 <br> G 01 N 33/543 <br> C 12 N 11/00 |
| Y | US-A-4 282 287  (R.W. GIESE) <br> * Das ganze Dokument * <br> — — — | 1 | C 12 N 11/06 |
| Y,D | NATURE, Band 301, Nr. 1, 13. Januar 1983, Seiten 125-129, MacMillan Journals Ltd, London, GB; E.E. UZGIRIS et al.: "Two-dimensional crystallization technique for imaging macromolecules, with application to antigen-antibodiy-complement complexes" <br> * Das ganze Dokument * <br> — — — — — | 1 | |

| | | | RECHERCHIERTE SACHGEBIETE (Int. Cl.5) |
|---|---|---|---|
| | | | C 12 Q <br> G 01 N <br> C 12 N |

Der vorliegende Recherchenbericht wurde für alle Patentansprüche erstellt

| Recherchenort | Abschlußdatum der Recherche | Prüfer |
|---|---|---|
| Den Haag | 22 Februar 91 | DOEPFER K-P. |